# EUROPÄISCHE PATENTSCHRIFT

(11) **EP 0 295 579 B1**
(45) Veröffentlichungstag und Bekanntmachung des Hinweises auf die Patenterteilung: **20.10.1993**
(21) Anmeldenummer: 88109243.1
(22) Anmeldetag: 10.06.1988
(51) Int. Cl.: A61B 17/30, A61B 17/38, B25B 9/02, A61C 3/10

(54) **Federzange oder Pinzette, insbesondere Koagulationspinzette**
Spring forceps or tweezer in particular coagulation tweezers
Pince à ressort ou pincette en particulier pincette de coagulation

(30) Priorität: 17.06.1987 DE 3720215; 04.07.1987 DE 3722142
(43) Veröffentlichungstag der Anmeldung: 21.12.1988
(73) Patentinhaber: USSC (Deutschland) GmbH, D-79798 Jestetten (DE)
(72) Erfinder: Spingler, Rolf, D-7891 Lottstetten-Nack (DE)
(74) Vertreter: Hiebsch, Gerhard F., Dipl.-Ing.

(56) Entgegenhaltungen:
- EP-A- 0 133 393
- EP-A- 0 159 453
- FR-A- 2 573 301
- US-A- 3 653 389

## Beschreibung

Die Erfindung betrifft eine Federzange oder Pinzette nach dem Oberbegriff des unabhängigen Patentanspruches.

Derartige Pinzetten sind zangenartige Instrumente mit federnden Armen zum Fassen und Halten; sie werden vornehmlich im chirurgischen oder zahntechnischen Bereich eingesetzt, wobei anatomische Pinzetten geriffelt, chirurgische Pinzetten gezähnt und Pinzetten zum Entfernen von Fremdkörpern mit feinen Spitzen versehen sind. Üblicherweise bestehen Pinzetten aus federnden Metallstreifen, welche im Bereich des als geschlossene Zone bezeichneten Pinzettenteiles aufeinanderliegen und dort miteinander verschweißt oder vernietet sind.

Ein chirurgisches Instrument gattungsgemäßer Art zeigt beispielhaft die EP-A-0 159 453 mit einem Handgriff aus weichem, heiß sterilisierbarem Werkstoff, der am Griffbereich des Instrumentes befestigt wird, beispielsweise durch einen Rastvorgang lösbar mit ihm verbindbar ist. Damit soll die Herstellung des Handgriffes preiswert sein und vor allem eine gute Griffigkeit auch bei Verwendung feuchter Gummihandschuhe anbieten, so daß beliebige -- unter Umständen auch große -- Kräfte übertragen werden können und bei einem Benutzer geringe Ermüdung eintritt.

Eine besondere Form chirurgischer Instrumente bilden die Koagulationspinzetten, deren Spitzen Strom zugeführt wird. Eine stromdurchflossene Pinzette ist in der FR-A-2 573 301 beschrieben.

Angesicht dieses Standes der Technik hat sich der Erfinder das Ziel gesetzt, eine eingangs erwähnte Federzange oder Pinzette zu schaffen, welche auf einfache Weise und kostengünstig herstellbar sowie von hoher Einsatzvielfalt ist. Ein besonderes Anliegen des Erfinders ist es, eine Einwegpinzette anzubieten.

Zur Lösung dieser Aufgabe führt die Lehre des unabhängigen Patentanspruches; die Unteransprüche geben weitere Ausgestaltungen an.

Bei der in ihrer Längsachse geteilten Pinzette weist erfindungsgemäß jedes Formteil eine Hälfte beider Pinzettenarme auf. Die Trennebene verläuft also innerhalb der Pinzettenarme und nicht -- wie dies vom Stande der Technik her bekannt -- im schlitzartigen Raum zwischen den Pinzettenarmen.

Die Formteile sind in besonders vorteilhafter Weise gleichgestaltet, so daß sowohl die Herstellung als auch die Lagerhaltung vereinfacht wird. Zudem hat es sich als günstig erwiesen, diese einzige Pinzettenhälfte aus Kunststoff herzustellen und anschließend beide Pinzettenhälften miteinander so zu verbinden, daß in den Pinzettenarmen erfindungsgemäß verlaufende und sich durch den gesamten Pinzettenkörper erstreckende Längsnuten in der fertigen Pinzette zu Kanälen ergänzt sind.

Die erfindungsgemäßen Kanäle erlauben es, gesonderte Greifspitzen am freien Ende der Pinzettenarme einzusetzen, auszuwechseln od. dgl. und somit die Einsatzvielfalt erheblich zu erhöhen.

Ein anderer Vorzug der erfindungsgemäßen Pinzette liegt darin, daß beispielsweise bei Koagulationspinzetten die an die Greifenden anschließenden Stromleitungen problemlos in den von den Längsnuten erzeugten Kanälen verlegt werden können - in einer besonders günstigen Ausführung enden diese Stromleiter an Steckerstiften, die ihrerseits wiederum jeweils in einem der Kanäle festgelegt sein können. Bevorzugt ist es auch möglich, Stränge aus elektrisch leitendem Werkstoff in diese Kanäle einzulegen, die endwärts einerseits eine Greifspitze sowie anderseits einen Kontaktstift bilden.

Es wird deutlich, daß die erfindungsgemäße Ausgestaltung der Pinzettenhälfte eine außergewöhnliche Vielfalt von Pinzettenvariationen erlaubt, dies bei geringer Lagerhaltung und einfacher Montagemöglichkeit.

Von Bedeutung für diese Montagevereinfachung ist ein von der Innenfläche der Pinzettenhälfte abragender Stift, dem spiegelbildlich auf der anderen Seite der Pinzettenlängsachse ein Sackloch entsprechenden Querschnittes zugeordnet ist; beim Zusammensetzen zweier Pinzettenhälften greifen die beschriebenen Teile ineinander und ermöglichen so ein schnelles Zentrieren, gegebenenfalls sogar einen festen Halt beider Pinzettenhälften aneinander.

Im Rahmen der Erfindung liegt auch eine besondere Art der Führung der Pinzettenarme beim Greifvorgang, d. h. beim Zueinanderführen der Pinzettenarme. Es handelt sich dabei um wenigstens ein Paar von Anformungen an die Pinzettenarmhälfte einer Pinzettenhälfte, wobei vorteilhafterweise von jedem ihrer Pinzettenarme eine solche Anformung in den schlitzartigen Raum einragt und zwar so, daß die Innenfläche der einen Anformung an der Innenfläche der anderen Anformung bei dem beschriebenen Greifvorgang entlanggleitet und so beim Schließen des Pinzettenmaules exakt führt. Von Bedeutung ist auch, daß dank der Gleichförmigkeit der Pinzettenhälften die beiden Anformungspaare an den Pinzettenseiten zueinander spiegelverkehrt liegen. Dies verbessert das Führungsvermögen erheblich.

Das Griffelement, an dem die Finger des Chirurgen oder sonstigen Benutzers greifen, besteht im vorliegenden Falle aus radialen Teilringrippen, die im geschlossenen Zustand des Pinzettenmaules Ringe bilden. Diese Teilringrippen entstehen durch die Einformung von Umfangsnuten in die Pinzettenarme. Die Rippenoberflächen sind durch Anformungen aufgerauht.

Es ergibt sich insgesamt ein einfacher Wegwerfartikel mit den weiter oben bereits beschriebenen Vorzügen.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in:
- Fig. 1:: die Draufsicht auf eine Pinzette aus zwei Pinzettenhälften;
- Fig. 2:: eine Seitenansicht zu Fig. 1 mit der Pinzette in Ruhelage;
- Fig. 3:: die Ansicht gemäß Fig. 2 mit Pinzette in Greifstellung;
- Fig. 4:: eine der Pinzettenhälften;
- Fig. 5:: den vergrößerten Querschnitt durch Fig. 3 nach deren Linie V - V;
- Fig. 6,7,8:: Stirnansichten gemäß Pfeil VI bzw. Pfeil VII in Fig. 2 bzw. Pfeil VIII in Fig. 4;
- Fig. 9:: eine geschnittene Teilschrägsicht.

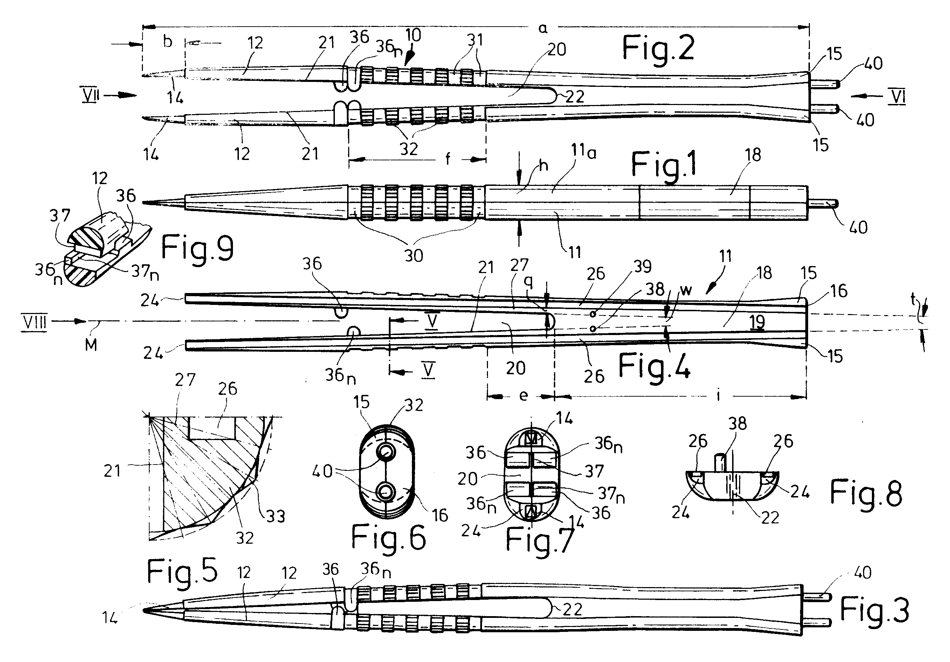

Eine Pinzette 10 einer beispielsweisen Länge a von 150 mm besteht -- wie insbesondere Fig. 1 verdeutlicht -- aus zwei gleichgeformten, mit ihren Innenflächen 19 aufeinanderliegenden Pinzettenhälften 11,11ₐ. Diese ergeben hier zusammen eine Pinzettenhöhe h von etwa 8 mm. Von der oben bezifferten Gesamtlänge a der Pinzette 10 nimmt die Kraglänge b zweier in Pinzettenarme 12 eingelegter Greifspitzen 14 etwa 10 mm ein.

Jede Pinsettenhälfte 11,11ₐ weist eine von einer -- eine flossenartige Erweiterung 15 anbietenden -- Stirnfläche 16 ausgehende geschlossene Zone 18 auf, deren Länge i etwa 60 mm beträgt und an welcher ein zwischen jenen Pinzettenarmen 12 verlaufender schlitzartiger Raum 20 endet. Dieser beginnt an den Stirnenden 24 der Pinzettenarme 12 und seine Schlitzkanten bzw. Pinzettenarmkanten 21 laufen bis zu seinem Schlitztiefsten 22 in einem spitzen Winkel w konisch zu. Der Konuswinkel w ist größer als ein Winkel t, den zwei den Schlitz 20 flankierende Längsnuten 26 miteinander konstruktiv begrenzen. Dies bedeutet, daß die Pinzettenarmkanten 21 im Bereich des Schlitztiefsten 22 von der jeweils benachbarten Längsnut 26 -- unter Bildung einer inneren Nutwandung 27 -- in einem Abstand q von beispielsweise 1,2 mm entfernt liegen und an den Stirnenden 24 nahezu unmittelbar -- entlang der hier sehr dünnen Nutwandung 27 -- an der Längsnut 26 verlaufen.

Etwa in einem Abstand e -- von hier etwa 16 mm -- vom Schlitztiefsten 22 ab beginnt eine Griffzone 30 einer Länge f von mehr als 30 mm; durch Umfangsnuten 31 werden Teilring-Rippen 32 mit eine Rändelung 33 ergebenden Anformungen dreieckförmigen Querschnitts erzeugt.

Am spitzennahen Ende jener Griffzone 30 ist in Fig. 3 an jedem Pinzettenarm 12 eine in den Schlitz 20 einragende noppenartige Anformung 36,36ₙ zu erkennen; beide Anformungen 36,36ₙ sind zueinander in Richtung der Pinzettenmittelachse M seitenversetzt. Die zu letzterer weisenden Innenfläche 37 der Anformung 36 berührt die benachbarte Innenfläche 37ₙ der anderen Anformung 36ₙ in der in Fig. 3 dargestellten Greifstellung, also bei zusammengedrückten Pinzettenarmen 12. Die Berührung der beschriebenen Anformungspaare 36/36ₙ ergibt eine sehr präzise Führung der Pinzettenarme 12 während des Greifvorganges.

Im übrigen ragt von der Innenfläche 19 -- außerhalb der Mittelachse M -- ein Stift 38 auf, dem auf der anderen Seite der Mittelachse M ein entsprechendes Sackloch 39 spiegelbildlich gegenüberliegt; beim Zusammenbau der Pinzettenhälften 11,11ₐ greift der Stift 38 der einen Hälfte in das Sackloch 39 der anderen Hälfte ein. Außerdem ergänzen sich die Längsnuten 26 dann zu Kanälen.

Es bedarf hier keiner weiteren Schilderung, daß in den aus den Längsnuten 26 entstehenden Kanälen der Pinzette 10 Spitzen 14 mit bezüglich des freien Endes beliebiger Querschnittsform festgelegt werden können. Von Bedeutung ist, daß diese Greifspitzen 14 undrehbar in den Längsnuten 26 ruhen, was durch einen polygonen Querschnitt geschehen kann oder durch Ein- bzw. Ausformungen in den Längsnuten, in welche dann Aus- bzw. Einformungen des Spitzensockels eingreifen.

Bei der dargestellten Pinzette 10, welche als Koagulationspinzette Verwendung findet, sind die Greifspitzen 14 an elektrische Kabel angeschlossen, die in den Kanälen 26 verlaufen und beispielsweise an Steckerstiften 40 enden, welche ihrerseits in den Kanälen 26 festliegen. Bei einer besonderen Ausführungsform ist jede Greifspitze 14 Teil eines Stabes aus elektrisch leitendem Werkstoff, der in den Kanal 26 eingelegt ist und als Steckerstift 40 endet.

Im Falle der Verwendung der Pinzette 10 als Einwegoperationspinzette entfallen naheliegenderweise die elektrischen Bestückungen.

## Patentansprüche

1. Federzange oder Pinzette mit einem Griffelement, insbesondere Koaqulationspinzette (10), mit zwei jeweils ein freies Greifende (14) aufweisenden, einen schlitzartigen Raum (20) begrenzenden, sowie federnd zueinanderführbaren Pinzettenarmen (12), die von einer gemeinsamen geschlossenen Zone (18) mit zumindest zwei diese in Längsrichtung unterteilenden Formteilen (11,11a) ausgehen, die mit ihren Innenflächen aneinanderliegend miteinander verbunden sind,
dadurch gekennzeichnet,
daß jeder Formteil (11 und 11a) eine Hälfte beider Pinzettenarme (12) aufweist.

2. Federzange oder Pinzette nach Anspruch 1, dadurch gekennzeichnet, daß die Formteile (11 und 11a) gleichgestaltet sind.

3. Federzange oder Pinzette nach Anspruch 1 oder 2, dadurch gekennzeichnet, daß die Formteile (11 und 11a) aus Kunststoff bestehen.

4. Federzange oder Pinzette nach einem der Ansprüche 1 bis 3, dadurch gekennzeichnet, daß in jedem Pinzettenarm (12) einer Formhälfte (11,11ₐ) eine Längsnut (26) verlauft, die beidseits in den Stirnflächen (16,24) der Pinzette (10) ausmündet, wobei gegebenenfalls die Längsnut (26) jedes Formteils (11,11ₐ) zumindest teilweise als abgedeckter Kanal ausgebildet ist.

5. Federzange oder Pinzette nach wenigstens einem der Ansprüche 1 bis 4, dadurch gekennzeichnet, daß in der Längsnut (26) ein elektrisch leitender Strang lagert, der einends als Greifspitze (14) und andernends als Steckerstift (40) aus der Federzange (10) ragt.

6. Federzange oder Pinzette mit in ihrer Ruhelage einen sich von den Greifenden verjüngenden schlitzartigen Raum nach Anspruch 4, dadurch gekennzeichnet, daß die beiden Längsnuten (26) miteinander einen Winkel (t) begrenzen, der größer ist als der Konuswinkel des schlitzartigen Raumes (20).

7. Federzange oder Pinzette nach wenigstens einem der Ansprüche 1 bis 6, dadurch gekennzeichnet, daß der Querschnitt der Längsnut (25,26) zumindest am Greifende (24) wenigstens eine Ecke und/oder Ein- bzw. Ausformungen aufweist.

8. Federzange oder Pinzette nach wenigstens einem der Ansprüche 1 bis 7, dadurch gekennzeichnet, daß die Dicke (q) der Nutenwandung (27) zwischen der Pinzettenarmkante (21) einerseits und der Längsnut (26) anderseits zum Greifende (24) des Pinzettenarmes (12) hin abnimmt.

9. Federzange oder Pinzette nach wenigstens einem der Ansprüche 1 bis 8, dadurch gekennzeichnet, daß von dem Pinzettenarm (12) jeder Formhälte (11ₐ) zumindest ein Paar von Anformungen (36,36ₙ) in den schlitzartigen Raum (20) ragt, wobei diese Anformungen in geschlossener Gebrauchsstellung der Pinzettenarme nebeneinander liegen bzw. sich aneinanderschmiegen und gegebenenfalls einander mit ihren Innenflächen (37,37ₙ) gegenüberstehen.

10. Federzange oder Pinzette nach wenigstens einem der Ansprüche 1 bis 9, dadurch gekennzeichnet, daß von der Innenfläche (19) des Formteils (11,11ₐ) zumindest ein Stift (38) od. dgl. aufragt, dem spiegelbildlich auf der anderen Seite der Pinzettenlängsachse (M) ein Sackloch (39) entsprechenden Querschnittes zugeordnet ist, und/oder daß jeder Pinzettenarm (12) mit einem Griffelement in Form von teilringförmigen Rippen (32) mit korrugierter Oberfläche (33) ausgestattet ist.

11. Federzange oder Pinzette nach wenigstens einem der Ansprüche 1 bis 4 und/oder 6 bis 10, dadurch gekennzeichnet, daß in die Längsnut (26) bzw. in den entsprechenden Kanal am Pinzettenarm (12) eine gesonderte Greifspitze (14) undrehbar eingesetzt ist.

12. Federzange oder Pinzette nach wenigstens einem der Ansprüche 1 bis 11, dadurch gekennzeichnet, daß in die Längsnut (25,26) bzw. in den entsprechenden Kanal am spitzenfernen Ende der Pinzette (10) ein elektrisches Kontaktelement (42) eingesetzt und dieses mit der Greifspitze (14) elektrisch verbunden ist.

## Claims

1. Spring forceps or tweezers comprising a gripping element, in particular coagulation tweezers (10), with two tweezer arms (12) each having a free gripping end (14), defining a slot-shaped space (20) and movable resiliently towards one another, these tweezer arms departing from a common closed zone (18) with at least two shaped parts (11, 11ₐ) which divide the latter in the longitudinal direction and are connected together so that their inner faces bear against one another, characterised in that each shaped part (11 and 11ₐ) contains half of the two tweezer arms (12).

2. Spring forceps or tweezers according to claim 1, characterised in that the shaped parts (11 and 11ₐ) are of identical design.

3. Spring forceps or tweezers according to claim 1 or claim 2, characterised in that the shaped parts (11 and 11ₐ) consist of plastic.

4. Spring forceps or tweezers according to one of claims 1 to 3, characterised in that a longitudinal groove (26) extends along each tweezer arm (12) of one half (11, 11ₐ) and opens at either end into the end faces (16, 24) of the tweezers (10), the longitudinal groove (26) of each shaped part (11, 11ₐ) optionally being designed at least partially as a covered channel.

5. Spring forceps or tweezers according to at least one of claims 1 to 4, characterised in that a conductive strand rests in the longitudinal groove (26) and projects from the spring forceps (10) at one end as a gripping point (14) and at the other end as a contact pin (40).

6. Spring forceps or tweezers with a slot-shaped space tapering from the gripping ends in its rest position according to claim 4, characterised in that the two longitudinal grooves (26) together define an angle (t) which is larger than the cone angle of the slot-shaped space (20).

7. Spring forceps or tweezers according to at least one of claims 1 to 6, characterised in that the cross section of the longitudinal groove (25, 26) has at least one corner and/or recesses or ribs at least at the gripping end (24).

8. Spring forceps or tweezers according to at least one claims 1 to 7, characterised in that the thickness (q) of the groove wall (27) between the tweezer arm edge (21), on the one hand, and the longitudinal groove (26), on the other hand, decreases towards the gripping end (24) of the tweezer arm (12).

9. Spring forceps or tweezers according to at least one of claims 1 to 8, characterised in that at least one pair of integrally moulded portions (36, 36ₙ) projects from the tweezer arm (12) of each half (11ₐ) into the slot-shaped space (20), these integrally moulded portions being situated alongside one another or fitting closely together and possibly lying opposite one another via their inner faces (37, 37ₙ) in the closed operating position of the tweezer arms.

10. Spring forceps or tweezers according to at least one of claims 1 to 9, characterised in that at least one pin (38) or the like projects from the inner face (19) of the shaped part (11, 11ₐ), a blind hole (39) of corresponding cross section being associated therewith in mirror image on the other side of the longitudinal axis (M) of the tweezers, and/or that each tweezer arm (12) is provided with a gripping element in the form of semi-circular ribs (32) with a corrugated surface (33).

11. Spring forceps or tweezers according to at least one of claims 1 to 4 and/or 6 to 10, characterised in that a separate gripping point (14) is inserted in a non-rotatable manner into the longitudinal groove (26) or into the corresponding channel on the tweezer arm (12).

12. Spring forceps or tweezers according to at least one of claims 1 to 11, characterised in that an electrical contact element (42) is inserted into the longitudinal groove (25, 26) or into the corresponding channel at the end of the tweezers (10) remote from the point and that this is electrically connected to the gripping point (14).

## Revendications

1. Pince élastique ou pincette possédant un élément poignée, notamment pincette de coagulation (10) comportant deux branches (12) de pincette qui présentent chacune une extrémité de préhension libre (14), qui délimitent un espace (20) en forme de fente, et qui peuvent être rapprochées élastiquement l'une de l'autre, qui partent d'une zone commune fermée (18) comportant elle-même au moins deux pièces de forme (11, 11ₐ) qui subdivisent cette zone dans la direction longitudinale et qui sont réunies entre elles, en appui par leurs surfaces intérieures, caractérisée en ce que chaque pièce de forme (11 ou 11ₐ) présente une moitié des deux branches (12) de la pincette.

2. Pince élastique ou pincette selon la revendication 1, caractérisée en ce que les pièces de forme (11 et 11ₐ) sont de même configuration.

3. Pince élastique ou pincette selon la revendication 1 ou 2, caractérisée en ce que les pièces de forme (11 et 11ₐ) sont en matière plastique.

4. Pince élastique ou pincette selon une des revendications 1 à 3, caractérisée en ce que, dans chaque branche (12) d'une moitié de forme (11, 11ₐ) de la pincette s'étend une rainure longitudinale (26) qui débouche des deux côtés dans les surfaces frontales (16, 24) de la pincette (10), la rainure longitudinale (26) de chaque pièce de forme formant éventuellement au moins partiellement un canal recouvert.

5. Pince élastique ou pincette selon au moins une des revendications 1 à 4, caractérisée en ce que, dans la rainure longitudinale (26) est disposé un élément conducteur d'électricité qui dépasse de la pince élastique (10) à une extrémité pour former une pointe de préhension (14) et à l'autre extrémité pour former une broche de connexion (40).

6. Pince élastique ou pincette selon la revendication 4, comportant, dans sa position de repos, un espace en forme de fente qui se rétrécit à partir des extrémités de prise, caractérisée en ce que les deux rainures longitudinales (26) forment entre elles un angle (t) qui est plus grand que l'angle de cône de l'espace (20) en forme de fente.

7. Pince élastique ou pincette selon au moins une des revendications 1 à 6, caractérisée en ce que la section transversale de la rainure longitudinale (25, 26) présente, au moins à l'extrémité de préhension (24), au moins un angle et/ou des formations rentrantes ou saillantes.

8. Pince élastique ou pincette selon au moins une des revendications 1 à 7, caractérisée en ce que l'épaisseur (q) de la paroi (27) de la rainure séparant le bord (21) de la branche de la pincette, d'une part, et la rainure longitudinale (26) d'autre part, décroît en direction de l'extrémité de préhension (24) de la branche (12) de la pincette.

9. Pince élastique ou pincette selon au moins une des revendications 1 à 8, caractérisée en ce que, sur la branche (12) de chaque moitié de forme (11ₐ) de la pincette, sont prévues au moins deux conformations (36, 36ₙ) qui font saillie dans l'espace (20) en forme de fente, et, dans la position d'utilisation fermée des branches de la pincette, ces conformations sont adjacentes l'une à l'autre ou sont serrées l'une contre l'autre, et le cas échéant se font face l'une à l'autre par leurs faces internes (37, 37ₙ).

10. Pince élastique ou pincette selon au moins une des revendications 1 à 9, caractérisée en ce que, sur la surface interne (19) de la pièce de forme (11, 11ₐ) fait saillie au moins une cheville (38) ou équivalent à laquelle est associé, de façon symétrique, de l'autre côté de l'axe longitudinal (M) de la pincette un trou borgne (39) de section correspondante, et/ou en ce que chaque branche (12) de la pincette est équipée d'un élément de préhension présentant la forme de nervures (32) en forme de parties d'anneaux munies d'une surface striée (33).

11. Pince élastique ou pincette selon au moins une des revendications 1 à 4 et/ou 6 à 10, caractérisée en ce qu'une pointe de préhension (14) distincte est insérée sans possibilité de rotation dans la rainure longitudinale (26) ou dans le canal correspondant de la branche (12) de la pincette.

12. Pince élastique ou pincette selon au moins une des revendications 1 à 11, caractérisée en ce qu'à l'extrémité de la pincette (10) qui est éloignée de la pointe, un élément de contact électrique (42) est inséré dans la rainure longitudinale (25, 26) ou dans le canal correspondant, et cet élément est relié électriquement à la pointe de préhension (14).
